# FASCICULE DE BREVET EUROPEEN

(11) **EP 1 786 484 B1**
(45) Date de publication et mention de la délivrance du brevet: **27.02.2008**
(21) Numéro de dépôt: 05778670.9
(22) Date de dépôt: 23.06.2005
(51) Int. Cl.: A61L 27/32, A61L 27/54

(54) **PROCEDE DE REVETEMENT D'UNE PROTHESE MEDICALE PAR UN OU PLUSIEURS AGENT(S) ANTIBACTERIEN(S)**
VERFAHREN ZUR BESCHICHTUNG EINER MEDIZINISCHEN PROTHESE MIT EINEM ODER MEHREREN ANTIBAKTERIELLEN AGENZIEN
METHOD FOR COATING A MEDICAL PROTHESIS WITH ONE OR SEVERAL ANTIBACTERIAL AGENTS

(30) Priorité: 02.07.2004 FR 0407365
(43) Date de publication de la demande: 23.05.2007
(73) Titulaire: Medical Coating, 69120 Vaulx En Velin (US)
(72) Inventeur: BIGNON, Aurélien, F-69001 Lyon (FR); VANDEVELDE, Richard, F-69140 Rillieux La Pape (FR)
(74) Mandataire: Maureau, Philippe
(86) Numéro de dépôt international: PCT/FR2005/001590
(87) Numéro de publication internationale: WO 2006/013244

(56) Documents cités:
- WO-A-03/035123

## Description

La présente invention concerne un procédé de revêtement d'au moins une partie de la surface d'une prothèse médicale par un ou plusieurs agent(s) antibactérien(s).

De nos jours, les revêtements de phosphate de calcium sur prothèses médicales sont reconnus pour leurs propriétés d'ostéoconduction, c'est à dire qu'ils favorisent la cicatrisation de l'os sur le revêtement si la prothèse est implantée en site osseux.

Cette ostéoconduction est liée à la composition des phosphates de calcium qui est proche de la composition de la partie minérale de l'os naturel. L'ostéoconduction est généralement améliorée par la porosité ou rugosité du revêtement ostéoconducteur, c'est pourquoi une sous-couche métallique ou céramique, poreuse ou rugueuse est parfois ajoutée sous la couche de phosphate de calcium.

Ces revêtements sont souvent réalisés à la surface de prothèses articulaires non cimentées pour favoriser une accroche « biologique » de la prothèse.

Les opérations chirurgicales font toujours courir un risque d'infection du site opératoire. Quelles que soient les précautions prises au bloc opératoire, des germes infectieux pénètrent dans la plaie au cours du geste chirurgical et peuvent se multiplier sur la prothèse ou dans l'os. Ces infections sont très dommageables pour le patient et sont difficiles à traiter.

Pour prévenir de telles infections, il est reconnu qu'il est nécessaire d'obtenir de fortes concentrations d'antibiotique au niveau du site opératoire pour avoir une action sur les germes qui peuvent être présents.

Pour les antibiotiques dose-dépendants (c'est-à-dire dont l'efficacité dépend de la dose en contact avec les germes), plus la dose est élevée, plus l'antibiotique est efficace, même si le temps de contact est court. Il est d'ailleurs nécessaire que le traitement antibiotique préventif soit le plus court possible (inférieur à 48 heures) afin d'éviter l'émergence de germes résistant à l'antibiotique.

Le moyen de prévention généralement utilisé par les chirurgiens est l'injection systémique (intraveineuse ou intramusculaire) d'un antibiotique.

Cette antibioprophylaxie systémique a l'avantage de protéger le patient pendant toute la durée de l'intervention chirurgicale (l'injection d'antibiotique est généralement réalisée un peu avant la première incision chirurgicale). Par contre, la plupart des antibiotiques ont une faible diffusion osseuse, si bien que la concentration d'antibiotique qui arrive au niveau du site opératoire n'est pas toujours efficace.

Il est donc avantageux d'obtenir un relargage local d'antibiotique dans le site opératoire pour améliorer efficacité de la prévention en augmentant la dose locale d'antibiotique, et compléter ainsi l'action de l'antibioprophylaxie systémique. L'antibiotique local et l'antibiotique systémique peuvent de plus avoir des spectres d'action complémentaires ou donner lieu à des synergies sur certains germes.

Pour les prothèses articulaires cimentées, il est possible d'utiliser des ciments acryliques contenant un antibiotique. Pour les prothsèes sans ciment, avec revêtement ostéoconducteur, il peut être intéressant d'effectuer un relargage local d'antibiotique à partir de la surface du revêtement ostéoconducteur. C'est l'objet de la présente invention.

Certains essais ont déjà été effectués, notamment en réalisant des revêtements antibactériens constitués d'un polymère résorbable et d'un agent antibactérien. La résorption du polymère ou la diffusion de l'agent antibactérien permettent son relargage dans l'environnement de la prothèse. Cependant, les cinétiques de résorption de ces polymères sont généralement assez lentes, si bien que si ces revêtements sont réalisés sur des prothèses à surface ostéoconductrice, le polymère va masquer la surface ostéoconductrice qui n'aura plus d'action.

Des essais ont aussi été réalisés en trempant la prothèse dans une solution antibiotique, juste avant l'implantation. Cependant, cette solution pose le problème du stockage de l'antibiotique sous forme liquide (généralement peu stable), séparément de la prothèse. De plus, le chirurgien est tenu de réaliser l'opération d'imprégnation au bloc opératoire, ce qui n'est pas très pratique et lui fait perdre du temps.

Si la prothèse est stockée dans la solution antibiotique, la solution peut modifier les propriétés du revêtement ostéoconducteur. De plus la dose d'antibiotique n'est pas du tout contrôlée puisqu'elle dépend de la masse de solution qui reste sur la prothèse après sa sortie de la solution antibiotique lors de l'implantation : si la dose d'antibiotique est trop forte, elle risque d'être toxique et si elle est trop faible elle ne sera pas efficace.

Des procédés ont donc été développés qui consistent à imprégner le revêtement ostéoconducteur avec une solution antibactérienne, puis à sécher afin d'éliminer le solvant et ne conserver à la surface de la prothèse que l'agent antibactérien. Cette méthode permet de conserver la prothèse prête à l'emploi pendant plusieurs années et de contrôler, pendant le processus de fabrication, la dose d'agent antibactérien incorporée. L'agent antibactérien est relargué en quelques heures, ce qui permet de libérer très vite la surface du revêtement ostéoconducteur qui peut alors permettre l'accroche biologique de la prothèse.

Un tel procédé est décrit dans la demande de brevet internationale PCT/US02/33675 déposée le 22 octobre 2002, et publiée le 1 mai 2003, sous le numéro WO 03/035123 A1.

Cette demande de brevet décrit un procédé dans lequel l'agent antibactérien est imprégné sur la prothèse par immersion de celle-ci dans une solution d'antibiotique dilué dans l'eau. Alternativement, la demanderesse propose une imprégnation de la solution antibiotique sur la prothèse, directement par le chirurgien en salle opératoire, par pipetage ou dépôt à l'aide d'une seringue.

Néanmoins, il est apparu que de telles méthodes d'imprégnation de l'antibiotique présentent l'inconvénient majeur de ne pas permettre un dosage précis de l'antibiotique réellement déposé sur la prothèse, et ceci, même lorsque cette opération est réalisée à l'aide d'une pipette ou d'une seringue. En effet, dans tous les cas se produit un ruissellement de la solution antibiotique, dont une certaine quantité n'imprègne pas la surface de la prothèse et est perdue. En outre, un autre désavantage majeur, des techniques d'imprégnation citées ci-avant, est la mauvaise répartition de la quantité d'antibiotique sur la surface traitée, ce qui peut modifier la cinétique de libération de l'antibiotique. De plus, l'aspect esthétique des prothèses est un argument commercial qui touche souvent les praticiens, or si l'antibiotique est inégalement réparti avant d'être séché, il va engendrer des taches peu esthétiques sur le revêtement.

La présente invention vise à résoudre les désavantages des procédés de traitement de prothèses décrits ci-avant, et à cet effet, elle propose un procédé de réalisation d'un revêtement actif sur au moins une portion de la surface d'une prothèse médicale, ledit revêtement actif comprenant un composé ostéoconducteur à base de phosphate de calcium et au moins un agent antibactérien, comprenant les étapes consistant, dans l'ordre, à:
- revêtir la portion de prothèse traitée par un composé ostéoconducteur à base de phosphate de calcium,
- décontaminer la prothèse,
- revêtir, en atmosphère propre, la portion de prothèse à traiter avec une solution comprenant au moins un agent antibactérien en solution ou en suspension dans un solvant,
- sécher la prothèse, puis la conditionner en emballage stérile,
caractérisé en ce que le revêtement par la solution antibactérienne est réalisé par pulvérisation de ladite solution sur la portion de prothèse à traiter, avant séchage de ladite prothèse.

Ainsi, grâce à la pulvérisation de l'agent antibactérien, celui-ci est réparti de manière homogène sur toute la surface de la prothèse à traiter, et les quantités pulvérisées sont très précisément contrôlées par le débit de pulvérisation, le temps de pulvérisation et la trajectoire de la buse de projection. La dose d'agent antibactérien déposée peut être facilement contrôlées par pesée de la prothèse avant pulvérisation et après séchage. En outre, du fait que la solution antibactérienne est distribuée sous forme d'aérosol, l'imprégnation par la surface de la prothèse est progressive et aucun ruissellement n'apparaît. Le procédé est facilement automatisable en embarquant la buse de projection sur un robot.

On choisira de manière appropriée le dispositif de pulvérisation pouvant convenir à la projection de l'agent antibactérien sur la prothèse, en contrôlant les quantités pulvérisées. Un tel dispositif ne sera pas décrit dans le cadre de la présente invention puisqu'il est connu en soi, et utilisé dans d'autres domaines d'application. Ce type de procédé se base généralement sur la passage de l'agent antibactérien sous pression à travers une buse. Un flux de gaz peut parfois être véhiculé dans la buse pour permettre l'atomisation du produit à pulvériser. D'autres procédés consistent à faire passer l'agent antibactérien sur une sonde ultrasonore permettant l'atomisation de celui-ci.

Avantageusement, l'agent antibactérien comprend au moins un agent antibiotique, de préférence choisi parmi la liste des : Aminosides (gentamicine, tobramycine, streptomycine, etc...), Pénicillines (Acide Clavulanique, Amoxicilline, etc...), Cephalosporines, Glycopeptides (vancomycine, etc...), Lincosamides (Clindamycine, etc...), et des combinaisons de ceux-ci.

Dans une variante préférée de l'invention, l'agent antibiotique est de la gentamicine et le solvant de l'eau.

En outre, le séchage est préférentiellement réalisé soit en étuve, soit sous flux d'air chaud.

Le composé ostéoconducteur est de préférence de l'hydroxyapatite.

Selon une variante d'exécution de l'invention, l'étape de revêtement de la portion de prothèse à traiter par un composé ostéoconducteur, est précédée d'une étape de revêtement de ladite portion par au moins une couche d'un matériau céramique ou métallique.

Dans une variante préférée de l'invention, le revêtement de composé ostéocondusteur à base de phosphate de calcium est réalisé par projection plasma.

Selon une première forme d'exécution de l'invention, la quantité de solvant par rapport à l'agent antibactérien est suffisamment faible pour que le mélange de solvant et d'agent antibactérien soit sous forme de poudre humide.

Dans une variante d'exécution alternative de l'invention, la projection d'au moins un agent antibactérien est réalisé en deux étapes par :
- pulvérisation sur la prothèse d'une poudre sèche d'agent antibactérien, puis
- pulvérisation sur la prothèse d'un solvant.

Dans ce cas, la prothèse et la poudre d'agent antibactérien sont préférentiellement polarisées avant pulvérisation de la poudre d'agent antibactérien.

Eventuellement, la pulvérisation du solvant de l'agent antibactérien se fait alors avant la pulvérisation de la poudre d'agent antibactérien.

Selon une dernière forme d'exécution de l'invention, la pulvérisation d'une solution comprenant au moins un agent antibactérien en solution ou en suspension dans un solvant, est réalisée simultanément à l'étape de projection plasma d'un composé de phosphate de calcium.

De préférence, la pulvérisation se fait par passage sous pression du produit à pulvériser à travers une buse de faible diamètre.

Egalement de préférence, un gaz passe dans la buse de pulvérisation, en même temps que le produit à pulvériser.

Alternativement, la pulvérisation se fait par passage du produit à pulvériser sur une sonde ultrasonore.

Pour sa bonne compréhension, l'invention va à présent être décrite en détail, à l'aide d'un exemple, donné à titre non limitatif, d'un procédé de traitement d'une prothèse par un ou plusieurs agent(s) antibactérien(s).

Le procédé de traitement d'une prothèse de hanche, selon l'invention, peut comprendre les étapes consistant, dans l'ordre, à :
- revêtir la tige de la prothèse par projection plasma - à haute température, c'est-à-dire une température supérieure à 10000°C - d'hydroxyapatite,
- décontaminer la prothèse ainsi revêtue pour la débarrasser de toute pollution physique ou biologique,
- en sortie du traitement de décontamination, faire rentrer la prothèse en atmosphère propre,
- réaliser une solution de gentamicine en dissolvant de la poudre de sulfate de gentamicine dans de l'eau pour préparation injectable, apyrogène et stérile,
- mettre la prothèse en rotation dans un montage approprié,
- pulvériser la solution de gentamicine sur la prothèse en la faisant passer dans une buse de projection embarquée sur un robot 6 axes (l'atomisation de la solution est obtenue en faisant passer dans la buse de projection, de l'air comprimé propre, en même temps que la solution de gentamicine),
- sécher la prothèse en étuve à 65°C pendant 30 minutes,
- conditionner la prothèse dans un emballage la protégeant des contaminations éventuelles
- stériliser la prothèse par irradiation gamma.

Il va de soi que l'invention n'est pas limitée à la forme de réalisation décrite ci-dessus à titre exemple mais qu'elle en embrasse au contraire toutes les variantes.

## Revendications

1. - Procédé de réalisation d'un revêtement actif sur au moins une portion de la surface d'une prothèse médicale, ledit revêtement actif comprenant un composé ostéoconducteur à base de phosphate de calcium et au moins un agent antibactérien, comprenant les étapes consistant, dans l'ordre, à:
- revêtir la portion de prothèse traitée par un composé ostéoconducteur à base de phosphate de calcium,
- décontaminer la prothèse,
- revêtir, en atmosphère propre, la portion de prothèse à traiter avec une solution comprenant au moins un agent antibactérien en solution ou en suspension dans un solvant,
- sécher la prothèse, puis la conditionner en emballage stérile,
**caractérisé en ce que** le revêtement par la solution antibactérienne est réalisé par pulvérisation de ladite solution sur la portion de prothèse à traiter, avant séchage de ladite prothèse.

2. - Procédé selon la revendication 1, **caractérisé en ce que** l'agent antibactérien comprend au moins un agent antibiotique.

3. - Procédé selon la revendication 2, **caractérisé en ce que** l'agent antibiotique est choisi parmi la liste des : Aminosides, Pénicillines, Cephalosporines, Glycopeptides, Lincosamides, et des combinaisons de ceux-ci.

4. - Procédé selon la revendication 2, **caractérisé en ce** l'antibiotique est de la gentamicine et le solvant de l'eau.

5. - Procédé selon les revendications 1 à 4, **caractérisé en ce que** le séchage est réalisé soit en étuve, soit sous flux d'air chaud.

6. - Procédé selon les revendications 1 à 5, **caractérisé en ce que** le composé ostéoconducteur est de l'hydroxyapatite.

7. - Procédé selon les revendications 1 à 6, **caractérisé en ce que** l'étape de revêtement de la portion de prothèse à traiter par un composé ostéoconducteur, est précédée d'une étape de revêtement de ladite portion par au moins une couche d'un matériau céramique ou métallique.

8. - Procédé selon les revendications 1 à 7, **caractérisé en ce que** le revêtement de composé ostéocondusteur à base de phosphate de calcium est réalisé par projection plasma.

9. - Procédé selon les revendications 1 à 7, **caractérisé en ce que** la pulvérisation d'une solution comprenant au moins un agent antibactérien en solution ou en suspension dans un solvant, est réalisée simultanément à l'étape de projection plasma d'un composé de phosphate de calcium.

10. - Procédé selon les revendications 1 à 8, **caractérisé en ce que** la quantité de solvant par rapport à l'agent antibactérien est suffisamment faible pour que le mélange de solvant et d'agent antibactérien soit sous forme de poudre humide.

11. - Procédé selon les revendications 1 à 8, **caractérisé en ce que** la projection d'au moins un agent antibactérien est réalisé en deux étapes par :
- pulvérisation sur la prothèse d'une poudre sèche d'agent antibactérien, puis
- pulvérisation sur la prothèse d'un solvant.

12. - Procédé selon la revendication 11, **caractérisé en ce que** la prothèse et la poudre d'agent antibactérien sont polarisées avant pulvérisation de la poudre d'agent antibactérien.

13. - Procédé selon la revendication 11, **caractérisé en ce que** la pulvérisation du solvant de l'agent antibactérien se fait avant la pulvérisation de la poudre d'agent antibactérien.

14. - Procédé selon les revendications 1 à 13, **caractérisé en ce que** la pulvérisation se fait par passage sous pression du produit à pulvériser à travers une buse de faible diamètre.

15. - Procédé selon la revendication 14, **caractérisé en ce qu'**un gaz passe dans la buse de pulvérisation, en même temps que le produit à pulvériser.

16. - Procédé selon les revendications 1 à 13, **caractérisé en ce que** la pulvérisation se fait par passage du produit à pulvériser sur une sonde ultrasonore.

## Claims

1. A method of producing an active coating on at least one portion of the surface of a medical prosthesis, said active coating comprising an osteoconductive compound based on calcium phosphate and at least one antibacterial agent, comprising Lhe steps consisting, in the following order, in:
- coating the treated prosthesis portion with an osteoconductive compound based on calcium phosphate,
- decontaminating the prosthesis,
- coating, under a clean atmosphere, the prosthesis portion to be treated with a solution comprising at least one antibacterial agent in solution or in suspension in a solvent,
- drying the prosthesis, then packaging in sterile packaging,
**characterised in that** coating with the antibacterial solution is effected by spraying said solution onto the prosthesis portion to be treated, prior to drying of said prosthesis.

2. A method according to claim 1, **characterised in that** the antibacterial agent comprises at least one antibiotic agent.

3. A method according to claim 2, **characterised in that** the antibiotic agent is selected from the list comprising: aminoglycosides, penicillins, cephalosporins, glycopeptides, lincosamides, and combinations thereof.

4. A method according to claim 2, **characterised in that** the antibiotic is gentamicin and the solvent is water.

5. A method according to claims 1 to 4, **characterised in that** drying is performed either in a cabinet or in a stream of hot air.

6. A method according to claims 1 to 5, **characterised in that** the osteoconductive compound is hydroxyapatite.

7. A method according to claims 1 to 6, **characterised in that** the step of coating the prosthesis portion to be treated with an osteoconductive compound is preceded by a step of coating said portion with at least one layer of a ceramic or metallic material.

8. A method according to claims 1 to 7, **characterised in that** the coating of osteoconductive compound based on calcium phosphate is produced by plasma spraying.

9. A method according to claims 1 to 7, **characterised in that** spraying of a solution comprising at least one antibacterial agent in solution or in suspension in a solvent is effected simultaneously with the step of plasma spraying of a calcium phosphate compound.

10. A method according to claims 1 to 8, **characterised in that** the quantity of solvent relative to the antibacterial agent is sufficiently low for the mixture of solvent and antibacterial agent to be in the form of moist powder.

11. A method according to claims 1 to 8, **characterised in that** spraying of at least one antibacterial agent is performed in two steps by:
- spraying a dry powder of antibacterial agent onto the prosthesis, then
- spraying a solvent onto the prosthesis.

12. A method according to claim 11, **characterised in that** the prosthesis and the antibacterial agent powder are polarised prior to spraying of the antibacterial agent powder.

13. A method according to claim 11, **characterised in that** spraying of the antibacterial agent solvent is performed prior Lo spraying of the antibacterial agent pcwdcr.

14. A method according to claims 1 to 13, **characterised in that** spraying is performed by passing the product to be sprayed through a small diameter nozzle under pressure.

15. A method according to claim 14, **characterised in that** a gas passes into the spraying nozzle at the same time as the product to be sprayed.

16. A method according to claims 1 Lo 13, **characterised in that** spraying is effected by passing the product to be sprayed over an ultrasound probe.

## Patentansprüche

1. Verfahren zur Herstellung einer aktiven Beschichtung auf mindestens einem Teil der Oberfläche einer medizinischen Prothese, wobei die aktive Beschichtung eine osteokonduktive Verbindung auf der Basis von Kalziumphosphat und zumindest einen antibakteriellen Wirkstoff enthält, das in der unten stehenden Reihenfolge folgende Schritte umfaßt:
• Beschichtung des behandelten Prothesenteils mit einer osteokonduktiven Verbindung auf der Basis von Kalziumphosphat,
• Dekontaminierung der Prothese,
• Beschichtung des zu behandelnden Prothesenteils mit einer Lösung, die zumindest einen antibakteriellen Wirkstoff in Lösung oder in Suspension in einem Lösungsmittel enthält, in sauberer Atmosphäre,
• Trocknung der Prothese, dann Verpackung in steriler Verpackung,
**dadurch gekennzeichnet, dass** die Beschichtung mit der antibakterielle Lösung durch Zerstäubung der Lösung auf dem zu behandelnden Prothesenteil erfolgt, bevor die Prothese getrocknet wird.

2. Verfahren nach Anspruch 1, **dadurch gekennzeichnet, dass** der antibakterielle Wirkstoff zumindest einen antibiotischen Wirkstoff enthält.

3. Verfahren nach Anspruch 2, **dadurch gekennzeichnet, dass** der antibiotische Wirkstoff aus der Liste der Aminoside, Penizilline, Cephalosporine, Glycopeptide, Lincosamide und deren Kombinationen miteinander gewählt ist.

4. Verfahren nach Anspruch 2, **dadurch gekennzeichnet, dass** das Antibiotikum Gentamincin und das Lösungsmittel Wasser ist.

5. Verfahren nach den Ansprüchen 1 bis 4, **dadurch gekennzeichnet, dass** die Trocknung entweder im Wärmeschrank oder unter Heißluftstrom erfolgt.

6. Verfahren nach den Ansprüchen 1 bis 5,**dadurch gekennzeichnet, dass** die osteokonduktive Verbindung Hydroxyapatit ist.

7. Verfahren nach den Ansprüchen 1 bis 6, **dadurch gekennzeichnet, dass** dem Schritt zur Beschichtung des zu behandelnden Prothesenteils mit einer osteokonduktiven Verbindung ein Schritt zur Beschichtung des Teils mit zumindest einer Schicht aus keramischem oder metallischem Material vorausgeht.

8. Verfahren nach den Ansprüchen 1 bis 7, **dadurch gekennzeichnet, dass** die Beschichtung mit der osteokonduktiven Verbindung auf der Basis von Kalziumphosphat durch Plasmaprojektion erfolgt.

9. Verfahren nach den Ansprüchen 1 bis 7, **dadurch gekennzeichnet, dass** die Zerstäubung einer Lösung, die zumindest einen antibakteriellen Wirkstoff in Lösung oder in Suspension in einem Lösungsmittel enthält, gleichzeitig mit dem Schritt der Plasmaprojektion einer Verbindung aus Kalziumphosphat erfolgt.

10. Verfahren nach den Ansprüchen 1 bis 8, **dadurch gekennzeichnet dass** die Menge an Lösungsmittel im Vergleich zu dem antibakteriellen Wirkstoff relativ gering ist, damit das Gemisch aus Lösungsmittel und aus antibakteriellem Wirkstoff die Form von feuchtem Pulver hat.

11. Verfahren nach den Ansprüchen 1 bis 8, **dadurch gekennzeichnet, dass** die Projektion mindestens eines antibakteriellen Wirkstoffs in zwei Schritten erfolgt, indem:
• auf der Prothese ein trockenes Pulver aus antibakteriellem Wirkstoff zerstäubt wird, und dann
• auf der Prothese ein Lösungsmittel zerstäubt wird.

12. Verfahren nach Anspruch 11, **dadurch gekennzeichnet, dass** die Prothese und das Pulver aus antibakteriellem Wirkstoff vor Zerstäubung des Pulvers aus antibakteriellem Wirkstoff polarisiert werden.

13. Verfahren nach Anspruch 11, **dadurch gekennzeichnet, dass** die Zerstäubung des Lösungsmittels des antibakteriellen Wirkstoffs vor der Zerstäubung des Pulvers aus antibakteriellem Wirkstoff erfolgt.

14. Verfahren nach den Ansprüchen 1 bis 13, **dadurch gekennzeichnet, dass** die Zerstäubung dadurch erfolgt, dass das zu zerstäubende Produkt durch eine Düse mit geringem Durchmesser hindurchgedrückt wird.

15. Verfahren nach Anspruch 14, **dadurch gekennzeichnet, dass** zum gleichen Zeitpunkt wie das zu zerstäubende Produkt ein Gas in die zerstäuberdüse eintritt.

16. Verfahren nach den Ansprüchen 1 bis 13, **dadurch gekennzeichnet, dass** die Zerstäubung dadurch erfolgt, dass das zu zerstäubende Produkt an einem Ultraschallsensor vorbeiläuft.
